# EUROPEAN PATENT SPECIFICATION

(11) **EP 3 168 617 B1**
(45) Date of publication and mention of the grant of the patent: **04.12.2019**
(21) Application number: 15757309.8
(22) Date of filing: 09.07.2015
(51) Int. Cl.: G01N 33/53, G01N 33/68

(54) **DETECTING GLUTEN PEPTIDES IN HUMAN FLUIDS**
DETEKTIERUNG DER PEPTIDEN VON GLUTEN IN MENSCHLICHEN FLÜSSIGKEITEN
DETECTION DES PEPTIDES DU GLUTEN DANS DES FLUIDES HUMAINS

(30) Priority: 09.07.2014 ES 201400569
(43) Date of publication of application: 17.05.2017
(73) Proprietor: Universidad de Sevilla, 41013 Sevilla (ES); Biomedal, S.L., 41092 Sevilla (ES)
(72) Inventor: MORENO AMADOR, María de Lourdes, 41013 Sevilla (ES); SOUSA MARTÍN, Carolina, 41013 Sevilla (ES); RODRÍGUEZ HERRERA, Alfonso, 41013 Sevilla (ES); CEBOLLA RAMÍREZ, Ángel, 41092 Sevilla (ES)
(74) Representative: Pons
(86) International application number: PCT/ES2015/070536
(87) International publication number: WO 2016/005643

(56) References cited:
- EP-A2- 2 660 593
- WO-A1-2013/098441
- WO-A2-2009/139887
- I. COMINO ET AL: "Monitoring of gluten-free diet compliance in celiac patients by assessment of gliadin 33-mer equivalent epitopes in feces", AMERICAN JOURNAL OF CLINICAL NUTRITION, vol. 95, no. 3, 1 March 2012 (2012-03-01), pages 670-677, XP055111731, ISSN: 0002-9165, DOI: 10.3945/ajcn.111.026708
- BELÉN MORÓN ET AL: "Toward the Assessment of Food Toxicity for Celiac Patients: Characterization of Monoclonal Antibodies to a Main Immunogenic Gluten Peptide", PLOS ONE, vol. 3, no. 5, 28 May 2008 (2008-05-28), page e2294, XP055074191, DOI: 10.1371/journal.pone.0002294
- Anonymous: "Body fluid - Wikipedia, the free encyclopedia", , 24 April 2013 (2013-04-24), XP055218909, Retrieved from the Internet: URL:https://web.archive.org/web/2013042420 2908/http://en.wikipedia.org/wiki/Body_flu id [retrieved on 2015-10-07]
- S. AURICCHIO: "An innovative approach to measure compliance to a gluten-free diet", THE AMERICAN JOURNAL OF CLINICAL NUTRITION, vol. 95, no. 3, 1 February 2012 (2012-02-01), pages 537-538, XP055218745, US ISSN: 0002-9165, DOI: 10.3945/ajcn.111.032888

## Description

### OBJECT OF THE INVENTION

The present invention relates to a process for detecting and quantifying gluten peptides resistant to gastrointestinal digestion in human body fluids, preferably urine, by means of immunological assays. This methodology is applicable in the medical-clinical sector, both for monitoring the adherence of coeliac patients to a gluten-free diet, patients with non-coeliac sensitivity to gluten, patients allergic to gluten and patients with any type of sensitivity to gluten proteins, and for accurately diagnosing refractory coeliac disease. The invention can also be applied in clinical research of coeliac disease to verify the effectiveness of future strategies related to the elimination or reduction of gluten toxicity.

### STATE OF THE ART

Coeliac disease (CD) is an autoimmune disorder caused by the ingestion of gluten proteins found in wheat, barley, rye and some oat varieties (Arent-Hansen et al., 2004; Kagnoff, 2007; Comino et al., 2011) which affects approximately 1% of the general population (Rubio-Tapia et al, 2009; Lionetti and Catassi, 2011; Bernardo and Peña, 2012; Sapone et al., 2012). The ingestion of gluten causes an inflammatory reaction in the upper part of the small intestine, which gives rise to tissue damage and villous atrophy.

Although most proteins in the diet are digested by gastrointestinal proteases to simple amino acids, dipeptides or tripeptides, gluten proteins are not fully digested and remain in the intestine (Ganapathy et al., 2006; Fasano 2009; Comino et al., 2012). These peptides are capable of internalisation into intestinal cells and, consequently, their glutamine residues can be deaminated by tissue transglutaminase (tTG). The deaminated peptides induce an immunological reaction, producing a reduction in intestinal absorption that can give rise to symptoms such as diarrhea, anaemia, retarded growth, weight loss, bone disorders, neurological complications, cancer, etc. (Alaedini and Green, 2005; Catassi and Fasano, 2008; Tack et al., 2010).

There is clear evidence supporting the majority contribution of epitopes related to, or contained in, the wheat alpha-2 gliadin-derived 33-mer peptide (57-89 residues) in the gluten immunotoxicity in coeliac patients. Gluten epitopes with high immunogenicity are located in regions of gliadins rich in proline and glutamine residues (Shan et al., 2002; Tye-Din et al., 2010; Comino et al., 2011; Real et al., 2012; Dessi et al., 2013).

Currently, strict adherence to a gluten-free diet (GFD) is the only effective treatment for CD and other cases of gluten intolerance. In most patients, strict adherence to GFD leads, in just a few months, to the fast and complete recovery of the normal architecture and function of small intestine mucosa, as well as the remission of the symptoms and normalisation of serological tests (Bernardo and Peña, 2012; Hall et al., 2013). Therefore, monitoring adherence in coeliac patients is of vital importance to avoid direct or indirect cumulative damages and to confirm that the persistence of any symptoms is not due to GFD infringement (voluntary or involuntary).

GFD monitoring entails a number of restrictions due to its social and economic implications. Approximately more than half of currently marketed foods contain what, barley, rye or oat gluten, including those in which it only participates as a thickener or binding agent (Freeman, 2013). GFD infringement has been associated with diarrheas, dyspepsia, osteoporosis, anaemia due to iron deficiency, depression and infertility, symptoms that disappear or improve, to a certain extent, by means of GFD adherence. In fact, strict GFD non-adherence can increase by 4.3 times the possibility of suffering lymphoma (Lebwohl and Green, 2003). These observations give us an idea of the importance of GFD adherence to reduce the symptoms, avoid nutritional deficiencies and improve the patient's quality of life. However, various studies based on intestinal biopsies suggest that at least one-third of CD patients do not fully adhere to GFD (Ciacci et al., 2002; Silvester and Rashid, 2007; Barratt et al, 2011; Matoori et al., 2013). In addition, between 36% and 55% of fully GFD-adherent patients do not achieve full histological remission, probably due to the involuntary ingestion of gluten (Stoven et al, 2012; Tio et al., 2012; Hall et al., 2013; Matoori et al., 2013). However, the exposure of these patients to gluten through the consumption of food labelled as safe but containing traces of gluten does not explain the high number of patients in which there is no full remission of the histological mucosal damage (Koning et al., 2013).

Likewise, part of the coeliac population does not seem to respond positively to GFD and suffer persistent or recurring malabsorption symptoms and villous atrophy. This population could be suspected of having refractory coeliac disease (RCD), a rare disease (approximately between 5%-10% of CD patients) that appears in patients without apparent positive response to GFD (Al-Toma et al., 2007; Freeman, 2009; Rubio-Tapia and Murray, 2010). Although RCD was described in patients with supposed total absence of gluten ingestion, the involuntary ingestion and hypersensitivity to a small amount of gluten can also trigger the symptoms inherent to the pathology.

There is a high percentage of coeliacs (approximately 90%) who claim to have gluten ingestion symptoms during the week (Lähdeaho et al., 2014). For example, in some cases of severe diarrhea there are doubts among medical professionals when identifying whether the causes stem from a severe infection or having ingested gluten. The only tool available in this last case is identifying the infectious agent, which can be complex given the amount of originator microorganisms and the price and time required to perform the microbiological analysis.

Markers are currently available for assessing GFD adherence by coeliac patients, which include the observation of symptomatic improvement, dietary interview and reduction or normalisation of specific antibodies for CD such as anti-tissue transglutaminase (anti-tTG) or anti-deamidated gluten peptides (ADG) (Dipper et al., 2009; Sharkey et al., 2013; Vallejo et al., 2013; Vives- Pi et al., 2013). However, there is no consensus as to the frequency of performance of controls or the best measures for evaluating said adherence, in addition to the fact that some of these methods have shown significant limitations (Bai et al., 2012). Control using anti-tTg or ADG antibodies as markers has been proposed; however, in general, they become negative one year after commencing GFD, in addition to not implying complete intestinal mucosal healing (Tursi et al, 2003; Sharkey et al., 2013). These types of markers are more useful for verifying GFD infringement than for verifying strict GFD adherence. Therefore, serological methods may not be sufficiently sensitive for detecting occasional dietary transgressions that prevent complete recovery (Kaukien et al., 2002; 2007; Tursi et al., 2006). Therefore, they are not a direct manner of demonstrating gluten ingestion in order to avoid harmful effects. In fact, the consequences of dietary transgressions can only be measured observing mucosal inflammation and/or villous atrophy, which would require performing intestinal biopsies and, consequently, placing the patient under anaesthetic, with the possible risks, discomfort and medical costs entailed.

A more direct measurement of gluten ingestion could provide valuable information in patient monitoring: the detection of GFD infringement prior to anatomical damage, the detection of involuntary ingestion and evaluation of the degree of adherence to the treatment during the initial period (after the diagnosis, when patients are less familiarised with the diet), providing easy and reliable confirmation of the results obtained. Therefore, a sensitive and reliable market for monitoring and detecting gluten ingestion would be a useful tool for correct GFD adherence and, probably, for accurately diagnosing RCD.

Earlier works have demonstrated that monoclonal antibodies anti-33-mer G12 and A1, obtained against the main α-gliadin immunogenic peptide, are very useful in the detection of toxic gluten peptides, both in clinical and food research (Morón et al., 2008a; 2008b; Ehren y col., 2009; Comino et al., 2012; Real et al., 2014). The determination of gluten peptides in faeces has recently been proposed as a strategy for directly verifying GFD adherence. Assays based on G12 and A1 antibodies have allowed the detection of gluten-derived peptides which are excreted in human faeces with certain correlation with the amount of gluten ingested (Comino et al., 2012). Monitoring of the gluten digested in faeces in a group of volunteers subjected to a strict GFD indicated that a single ingestion of gluten can be detected by measuring said reactive peptides in faeces, determining their excretion between 2-4 days, without the additional ingestion of gluten.

Some extra-intestinal symptoms of CD such as dermatitis herpetiformis, osteoporosis, neurological disorders, etc., are difficult to explain without assuming the presence of peptides in the blood after gluten ingestion in coeliac patients (Chirdo et al., 1998; Stern et al., 2001; Riestra, 2008; Ludvigsson and Green, 2011). Therefore, the most convenient tools for ascertaining GFD infringement are those that directly detect the presence of immunogenic gluten peptides in human samples, as the presence of these peptides in a coeliac patient would be the most direct and unequivocal way of determining whether the individual has ingested gluten or not.

Document WO 2009/139887 discloses a method for monitoring gluten ingestion by means of detecting gluten peptides in faeces using antibodies specific to immunogenic peptides resistant to gastrointestinal digestion.

Patent EP 2660593 develops biomarkers for Celiac Sprue which are peptide analogues of native gluten peptides. Said analogs are detected in urine.

To date, we have scarce direct resources for controlling GFD adherence in these patients. The detection of gluten peptides in faeces has the drawback that handling the samples is awkward and they are detected only 24 hours after ingestion and require an initial peptide extraction process.

Akobeng AK, Thomas AG. 2008. Systematic review: tolerable amount of gluten for people with coeliac disease. Aliment Pharmacol Ther; 27:1044-1052.

Alaedini A, Green P. 2005. Narrative review: coeliac disease: understanding a complex autoimmune disorder. Am. College Phys; 142:289-298.

Al-toma A, Verbeek WH, Mulder CJ..2007. The management of complicated coeliac disease. Dig Dis; 25:230-236.

Arentz-Hansen H, Fleckenstein B, Molberg Ø, Scott H, Koning F, Jung G, Roepstorff P, Lundin KE, Sollid LM. 2004. The molecular basis for oat intolerance in patients with coeliac disease. PLoS Medic; 1:84-92.

Bai J, Zeballos E, Fried M, Corazza GR, Schuppan D, Farthing MJG, Catassi C, Greco L, Cohen H, Krabshuis JH. 2012. WGOOMGE Practice Guideline Coeliac Disease. World Gastroenterol News; 2:S1-S8.

Barratt SM, Leeds JS, Sanders DS. 2011. Quality of life in coeliac disease is determined by perceived degree of difficulty adhering to a gluten-free diet, not the level of dietary adherence ultimately achieved. J Gastrointest Liver Dis; 20:241-245.

Bernardo D, Peña AS. 2012. Developing strategies to improve the quality of life of patients with gluten intolerance in patients with and without coeliac disease. Eur J Intern Med; 23:6-8.

Catassi C, Fabiani E, Iacono G, D'Agate C, Francavilla R, Biagi F, Volta U, Accomando S, Picarelli A, De Vitis I, et al. 2007. A prospective, double blind, placebo-controlled trial to establish a safe gluten threshold for patients with coeliac disease. Am J Clin Nutr; 85:160-166.

Catassi C, Fasano A. 2008. Coeliac disease. Curr Opin Gastroenterol; 24:687-691.

Ciacci C, Cirillo, M, Cavallaro R, Mazzacca G. 2002. Long-term follow-up of coeliac adults on gluten-free diet: prevalence and correlates of intestinal damage. Digestion; 66:178-185.

Comino I, Real A, de Lorenzo A, Cornell H, López-Casado MA, Barro F, Lorite P, Torres MI, Cebolla A, Sousa C. 2011. Diversity in oat potential immunogenicity: basis for the selection of oat varieties with no toxicity in coeliac disease. Gut; 60:915-922.

Comino I, Real A, Vivas S, Síglez MÁ, Caminero A, Nistal E, Casqueiro J, Rodriguez-Herrera A, Cebolla A, Sousa C. 2012. Monitoring of gluten-free diet compliance in coeliac patients by assessment of gliadin 33-mer equivalent epitopes in feces. Am J Clin Nutr; 95:670-677.

Dessì M, Noce A, Vergovich S, Noce G Daniele N. 2013. Safety Food in Coeliac Disease Patients: A Systematic Review. Food and Nutrition Sciences; Vol. 4 No. 7A:55-74.

Dipper CR, Maitra S, Thomas R, Lamb C A, McLean-Tooke A P, Ward R, Smith D, Spickett G, Mansfield JC. 2009. Alimentary Pharmacology & Therapeutics. Anti-tissue Transglutaminase Antibodies in the Follow-up of Adult Coeliac Disease. Aliment Pharmacol Ther; 30:236-244.

Ehren J, Moron B, Martin E, Bethune MT, Gray GM, Khosla C. 2009. A food-grade enzyme preparation with modest gluten detoxification properties. PLoS One; 21:e6313.

Esteban M, Castaño A. 2009. Non-invasive matrices in human biomonitoring: a review. Environ Int; 35:438-449.

Fasano A. 2009.Surprises from coeliac disease. Sci Am; 301:54-61.

Freeman HJ. 2009. Adult coeliac disease and its malignant complications. Gut Liver; 3:237-246.

Freeman HJ. 2013. Non-dietary forms of treatment for adult coeliac disease. World J Gastrointest Pharmacol Ther; 4:108-112.

Ganapathy V, Gupta N, Martindale RG. Physiology of the gastrointestinal tract. 4th ed. New York, NY: Johnson LR, 2006.

Gibert A, Kruizinga A G, Neuhold S, Houben G F, Canela M A, Fasano A, Catassi C. 2013. Might gluten traces in wheat substitutes pose a risk in patients with coeliac disease? A population-based probabilistic approach to risk estimation. Am J Clin Nutr; 97:109-116.

Hall NJ, Rubin GP, Charnock A. 2013. Intentional and inadvertent infringement in adult coeliac disease. A cross-sectional survey. Appetite; 68:56-62.

Hischenhuber C, Crevel R, Jarry B, Mäki M, Moneret-Vautrin DA, Romano A, Troncone R, Ward R. 2006. Review article: safe amounts of gluten for patients with wheat allergy or coeliac disease. Aliment Pharmacol Ther; 23:559-575.

Kagnoff F. 2007. Overview and pathogenesis of coeliac disease. Gastroenterology, 128:S10-S18.

Kaukinen K, Peraaho M, Lindfors K, et al. 2007. Persistent small bowel mucosal villous atrophy without symptoms in coeliac disease. Aliment Pharmacol Ther; 25:1237-1245.

Kaukinen K, Sulkanen S, Maki M, et al. 2002. IgA-class transglutaminase antibodies in evaluating the efficacy of gluten-free diet in coeliac disease. Eur J Gastroenterol Hepatol; 14:311-315.

Koning F, Mol M, Mearin M L. 2013. The million-dollar question: is "gluten-free" food safe for patients with coeliac disease? Am J Clin Nutr; 97:3-4.

Lähdeaho ML, Kaukinen K, Laurila K, Vuotikka P, Koivurova OP, Kärjä-Lahdensuu T, Marcantonio A, Adelman DC, Mäki M. 2014. Glutenase ALV003 Attenuates Gluten-Induced Mucosal Injury in Patients With Coeliac Disease. Gastroenterology; 146:1649-1658.

Lebwohl B, Green PH. 2003. Screening for coeliac disease. N Engl J Med; 349:1673-1674.

Li PKT, Burdmann EA, Mehta RL. 2013. Acute kidney injury: Global health alert. Transplantation; 5:653-657.

Lionetti E, Catassi C. 2011. New clues in coeliac disease epidemiology, pathogenesis, clinical manifestations, and treatment. Int Rev Immunol; 30:219-231.

Ludvigsson JF, Green PH. 2011. Clinical management of coeliac disease. J Intern Med; 269: 560-571.

Matoori S, Fuhrmann G, Leroux JC. 2013. Coeliac disease: a challenging disease for pharmaceutical scientists. Pharm Res; 30:619-626.

Moron B, Bethune MT, Comino I, Manyani H, Ferragud M, Lopez MC, Cebolla A, Khosla C, Sousa C. 2008a. Toward the assessment of food toxicity for coeliac patients: characterization of monoclonal antibodies to a main immunogenic gluten peptide. PLoS One 3:e2294.

Morón B, Cebolla A, Manyani H, Alvarez-Maqueda M, Megías M, Thomas M del C, López MC, Sousa C. 2008b. Sensitive detection of cereal fractions that are toxic to coeliac disease patients by using monoclonal antibodies to a main immunogenic wheat peptide. Am J Clin Nutr; 87:405-414.

Pinches M, Betts C, Bickerton S, Burdett L, Thomas H, Derbyshire N, Jones H B, Moores M. 2012. Evaluation of novel renal biomarkers with a cisplatin model of kidney injury: gender and dosage differences. Toxicol Pathol; 40:522-533.

Osman A. et al. 2001. A monoclonal antibody that recognizes a potential coeliac-toxic repetitive pentapeptide epitope in gliadins. European Journal of Gastroenterology & Hepatology; 13:1189-1193.

Pisitkun T, Johnstone R, Knepper MA. 2006. Discovery of urinary biomarkers. Mol Cell Proteomics; 5:1760-1771.

Real A, Comino I, de Lorenzo L, Merchán F, Gil-Humanes J, Giménez MJ, López-Casado MÁ, Torres MI, Cebolla Á, Sousa C, Barro F, Pistón F. 2012. Molecular and immunological characterization of gluten proteins isolated from oat cultivars that differ in toxicity for coeliac disease. PLoS One; 7:e48365. Real A, Comino I, Moreno ML, López - Casado MA, Lorite P, Torres MI, Cebolla A, Sousa C. 2014. Identification and in vitro reactivity of coeliac immunoactive peptides in an apparent gluten-free beer. PLoS One. 6: e100917.

Riestra, S. 2008. Enfermedades asociadas. Polanco I. (ed.) en Libro blanco de la Enfermedad Coeliaca, Madrid, España, pp. 41-49.

Rubio-Tapia A, Kyle RA, Kaplan E L, Johnson DR, Page W, Erdtmann F, Brantner TL, Kim WR, Phelps TK, Lahr BD, Zinsmeister AR, Melton LJ, Murray JA. 2009. Increased prevalence and mortality in undiagnosed coeliac disease. Gastroenterology; 137:88-93.

Rubio-Tapia A, Murray JA. 2010. Classification and management of refractory coeliac disease. Gut; 59:547-557.

Sapone A, Bai JC, Ciacci C, et al. 2012. Spectrum of gluten-related disorders: consensus on new nomenclature and classification. BMC Med; 10:13.

Shan L, Molberg Ø, Parrot I, Hausch F, Gray G M, Sollid L M, Khosla C. 2002. Structural basis for gluten intolerance in coeliac sprue. Science; 297:2275-2279.

Sharkey L M, Corbett G, Currie E, Lee J, Sweeney N, Woodward J M. 2013. Optimising delivery of care in coeliac disease-comparison of the benefits of repeat biopsy and serological follow-up. Aliment Pharmacol Ther; 38:1278-1291.

Silvester JA, Rashid M. 2007. Long-term follow-up of individuals with coeliac disease: an evaluation of current practice guidelines. Can J Gastroenterol; 21:557-564.

Stern M, Ciclitira PJ, Van Eckert R, Feighery C, Janssen FW, Mendez E, Mothes T, Troncone R, Wieser H. 2001. Analysis and clinical effects of gluten in coeliac disease. Eur J Gastroenterol Hepatol; 13:741-747.

Stoven S, Murray JA, Marietta E. 2012. Coeliac disease: advances in treatment via gluten modification. Clin Gastroenterol Hepatol; 10:859-862.

Tack GJ, Verbeek WH, Schreurs MW, Mulder CJ. 2010. The spectrum of coeliac disease: epidemiology, clinical aspects and treatment. Nat Rev Gastroenterol Hepatol; 7:204-213.

Tio M, Cox MR, Eslick GD. 2012. Meta-analysis: coeliac disease and the risk of all-cause mortality, any malignancy and lymphoid malignancy. Aliment Pharmacol Ther; 35:540-551.

Tursi A, Brandimarte G, Giorgetti GM. 2003. Lack of usefulness of anti-transglutaminase antibodies in assessing histologic recovery after gluten-free diet in coeliac disease. J Clin Gastroenterol; 37:387-391.

Tursi A, Brandimarte G, Giorgetti G M et al. 2006. Endoscopic and histological findings in the duodenum of adults with coeliac disease before and after changing to a glutenfree diet: a 2-year prospective study. Endoscopy; 38:702-707.

Tye-Din JA, Stewart JA, Dromey JA, Beissbarth T, van Heel DA, Tatham A, Henderson K, Mannering SI, Gianfrani C, Jewell DP, et al. 2010. Comprehensive, quantitative mapping of T cell epitopes in gluten in coeliac disease. Sci Transl Med; 2:41ra51.

Vallejo-Diez S, Bernardo D, Moreno Mde L, Muñoz-Suano A, Fernandez-Salazar L, Calvo C, Sousa C, Garrote JA, Cebolla A, Arranz E. 2013. Detection of specific IgA antibodies against a novel deamidated 8-mer gliadin peptide in blood plasma samples from coeliac patients. PLoS One; 22:e80982.

Vives-Pi M, Takasawa S, Pujol-Autonell I, Planas R, Cabre E, Ojanguren I, Montraveta M, Santos AL, Ruiz-Ortiz E. 2013. Biomarkers for diagnosis and monitoring of coeliac disease. J Clin Gastroenterol; 47:308-313.

### DESCRIPTION OF THE INVENTION

Urine samples are highly attractive due to the ease with which the sample is obtained, non-invasiveness, simple handling, easy homogenisation, low cost and easy transport and storage. Peptides and small proteins (< 10 kDa) are freely filtered by the glomerulus. However, there are few studies on low-molecular-weight peptides or protein fragments contained in urine due to the technical difficulty of measuring the peptides in the presence of proteins in diluted samples and the wide range of peptides expected.

The present invention indicates how gluten immunogenic peptides (GIP) can be detected in urine of coeliac individuals, through the concentration of peptides in urine and detection using anti-33-mer immunochromatographic strips, applicable in GFD clinical and monitoring studies and also in RCD diagnosis.

The present invention discloses a process for the non-invasive monitoring of gluten-free diet adherence through the detection and quantification of immunogenic gluten peptides resistant to gastrointestinal digestion in urine. Said immunogenic gluten-derived peptides resistant to intestinal degradation comprise the gluten peptide epitopes detected by the antibodies used in immunological assays. Therefore, the presence or absence of immunogenic gluten peptides resistant to intestinal enzymatic degradation is detected by immunological assays based on reactive antibodies directed thereagainst. Reactivity towards such peptides can be signalled by a variety of immunological techniques, including immunochromatography, biosensors, ELISA assays, detection using magnetic particles, etc. If the human body fluid has little concentration of gluten peptides, the process may require the concentration of immunogenic peptides by the passage of the fluid through columns as a previous step to the immunological assay. This methodology is applicable in the medical-clinical sector, both for verifying gluten-free diet adherence in coeliac patients, patients with gluten allergy or in patients with any kind of sensitivity to gluten proteins, and in the accurate diagnosis of refractory coeliac disease (RCD), i.e. in those cases where the symptoms persist despite the supposed total absence of gluten ingestion. The invention is also applicable to clinical CD research to verify the effectiveness of future strategies related to the elimination or reduction of gluten toxicity.

The object of the present invention is the application of immunological techniques based on antibodies that recognise immunogenic gluten-derived peptides resistant to intestinal degradation in samples of human body fluids. The sample is urine for detecting peptides as it allows the obtainment of larger volumes than others, such as saliva, sweat, serum, etc., thereby facilitating the work involved in collecting the samples. The preferred application of the invention is the detection of gluten ingestion in patients who are diagnosed and monitored due to having some degree of gluten sensitivity. It is preferably applicable to the verification of gluten ingestion during diagnosis of CD, verification of GFD adherence of diagnosed coeliac patients and to the detection of a case of potential gluten intoxication in any patient with immunotoxic gluten sensitivity. The invention is based on the use of immunological techniques that use antibodies that recognise immunotoxic gluten peptides resistant to gastrointestinal digestion.

In one aspect, the present invention relates to an *in vitro* process for detecting and/or quantifying the gluten ingested by a human being, hereinafter "method of the invention", which comprises bringing a biological fluid from said human being into contact with at least one antibody that specifically recognises gluten protein epitopes selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or their deaminated derivatives, wherein the recognition of the epitopes by the antibodies is indicative that the human being has ingested gluten.

In a particular embodiment, the amount of epitopes recognised by the antibody with respect to a control is indicative of the amount of gluten ingested and/or the time elapsed since gluten ingestion. As it will be understood by a person skilled in the art, the control will be defined, for example, by the technique quantification limit (QL), which can be calculated by means of a calibration curve or line using a pattern (such as the known amounts of a purified peptide containing epitopes recognised by the antibody), as shown in the examples accompanying this description (see Example 1). It is a standard of practice of a person skilled in the art to establish said QL to ascertain the amount of epitopes recognised by the antibody. As indicated previously, said epitopes are comprised within immunotoxic gluten peptides resistant to gastrointestinal digestion.

In the present invention, "biological fluid" is understood to be urine.

The term "antibody" relates to immunoglobin molecules or active portions of immunoglobin molecules, i.e. to molecules containing an antigen-binding site that is specifically bonded (immunoreacts) thereto. In the present invention, the antigen is any of the immunotoxic gluten peptides resistant to gastrointestinal digestion (hereinafter, "peptides of the invention") comprising the epitopes detected by the antibodies. Examples of portions of immunologically active immunoglobin molecules include fragments F(ab) and F(ab')2, which may be generated, for example, in a non-limiting manner, by treating the antibody with an enzyme such as pepsin. Another example of selection of the active portions of immunoglobins known in the state of the art is performed by means of genetic engineering techniques using the variable regions of immunoglobulins.

The antibody of the invention may be polyclonal (typically including different antibodies aimed at determinants or different epitopes) or monoclonal (aimed at a single determinant in the antigen). The expression "monoclonal antibody" alludes to a population of antibody molecules containing only one species of an antigen-binding site capable of immunoreacting with a particular epitope of the antigen. However, as known to a person skilled in the art, it should be noted that if the epitopes are very similar and are interrelated, the monoclonal antibody would be capable of recognising various epitopes.

The monoclonal antibody may be biochemically altered by means of genetic manipulation or may be synthetic, whereupon the antigen may possibly lack parts or all of the portions not required for recognising the peptide of the invention, being replaced by others that communicate additional advantageous properties to the antibody.

The antibody of the invention may be chimeric. Thus, a region of the heavy and/or light chain is identical or equivalent to the corresponding sequences in antibodies of a certain species or belonging to a class or subclass of certain antibodies, while the remaining chain(s) is/are identical or equivalent to the corresponding sequences in antibodies derived from other species or belonging to another class or subclass of antibodies, and fragments of said antibodies, such that they exhibit the desired reactivity.

The process of the invention comprises at least one antibody that specifically recognises the epitopes of gluten proteins selected from the group consisting of SEQ ID NO.: 2, SEQ ID NO.: 3, SEQ ID NO.: 4, SEQ ID NO.: 5, SEQ ID NO.: 6, SEQ ID NO.: 7, SEQ ID NO.: 8, SEQ ID NO.: 9, SEQ ID NO.: 10, SEQ ID NO.: 11 and/or their deaminated derivatives. Examples of antibodies that could be used in the context of the present invention include, but are not limited to, A1 and G12 monoclonal antibodies, which have been obtained by reactivity towards wheat alpha-gliadin 33-mer, although other antibodies that recognise peptides resistant to gluten digestion such as R5 or omega-gliadin anti-8-mer antibodies could also be used.

In a particular embodiment, the antibodies that specifically recognise gluten protein epitopes whose amino acid sequence comprises at least one-third of prolines and one-third of glutamins are selected from the group consisting of the monoclonal antibody G12, the monoclonal antibody A1 and the monoclonal antibody R5. These and other monoclonal antibodies that can be used for putting the present invention into practice are commercially available. The A1 antibody is commercially available from Biomedal, S.L. as the anti-gliadin antibody 33-mer AB-4747. The G12 antibody is commercially available from Biomedal, S.L. as the anti-gliadin antibody 33-mer AB-4748. The R5 antibody is commercially available under the brands R7001: RIDASCREEN® Gliadin (R-Biopharm), R7002: RIDASCREEN®FAST Gliadin (R-Biopharm), R7021: RIDASCREEN® Gliadin competitive (R-Biopharm), INgezim Gluten (Ingenasa).

The detection and/or quantification of peptides in human body fluids can be carried out, for example, but not limited to, immunological assays, immunochromatography using lateral flow strips, immunoprecipitation, immunomagnetic separation, protein arrays, immunofluorescence by detection by fluorescent particles in flow cytometry, electrochemical biosensors, electrochemical plasmon resonance biosensors, (for example, BIACORE®™), thermoelectric, nanomechanical and optical biosensors and, in short, any known immunological technique that is sufficiently sensitive and specific to guarantee the detection and quantification of gluten peptides.

Thus, in a particular embodiment, contact between the biological fluid and the antibodies is performed by means of an immunological method selected from the group consisting of immunochromatographic strips, fluorescent immunomicroparticles, magnetic immunoparticles, indirect ELISA, competitive ELISA, sandwich ELISA, Western blot, electrochemical biosensors, plasmon resonance biosensors, thermoelectric biosensors and nanomechanical biosensors. In a particular embodiment, the signal of the immunochromatographic strip is quantified by means of a reader.

A preferred manner of detecting gluten peptides could be using lateral flow tests with immunochromatography. The process of the invention should ideally allow the detection in urine of a specific gluten ingestion equivalent to 50 mg of wheat gluten per day, which is an amount situated in the maximum range described for a GFD or, where applicable, a minimum of 6.48 ng of gluten immunogenic peptides (GIP)/ml of urine.

A preferred object of the present invention are immunological analytical kits or devices based on antibodies reactive towards gluten peptides found in urine, which have been proven to be resistant to gastric digestion and immunogenics. These peptides could probably cross into the bloodstream and subsequently be excreted; including the peptides recognised by A1 and G12 antibodies.

The use of said analytical processes and kits to reveal the consumption of gluten, whether by contamination of the consumed foods, by malpractice in the handling of foods or by the occasional voluntary/involuntary ingestion of foods containing gluten, is also an object of the invention. Another object of the present invention is the application of the detection of said immunotoxic peptides in urine samples for CD clinical research purposes to verify the effectiveness of future therapies related to the reduction or elimination of gluten toxicity, which include enzymatic therapies with prolyl endopeptidases, the elimination of prolamins from the gluten of cereal grains, sequestrant immunotoxic peptide therapies, the detoxification of harmful gluten peptides in processed foods and other alternative therapies.

On the other hand, the present invention also envisages the use of immunological techniques to quantify the detection of gluten in human biological fluids, such as urine. As mentioned earlier, the immunological methods may be immunochromatographic strips, fluorescent immunomicroparticles, Western blots, ELISAs, biosensors based on electrochemical reactions catalysed by enzymes attached to the antibodies, antibody-coated magnetic particles, surface plasmon resonance, other optical biosensors, thermoelectric biosensors or any other method of those existing in the state of the art where through the concentration of an analyte (peptides) can be measured by an antibody.

Therefore, as mentioned earlier, these methods are characterised in that they use at least one antibody capable of detecting epitopes containing at least one-third of prolines and one-third of glutamins in their sequence, such as in the following sequences: SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and random substitutions of glutamate for glutamine in these peptides, which could occur by deamination mediated by transglutaminases. In a preferred embodiment of the invention, the antibodies used by immunological techniques would be G12 and, by extension, A1, due to their proven specifity and sensitivity. The invention also envisages the use of other antibodies such as R5, which is normally used to measure gluten in foods and which reacts with epitopes similar to the SEQ ID NO: 9 sequence that can also be found in gluten peptides resistant to gastrointestinal digestion which, although with less specifity and sensibility than peptides similar to the 33-mer peptide, may have sufficient sensitivity since they react with peptides resistant to proteases typical of cereal prolamins which are toxic to coeliac patients.

A preferred embodiment of the invention would be the use of immunochromatographic strips based on alpha-gliadin anti-33-mer antibodies, G12 and A1 antibodies, which allow semi-quantitative and fast detection of gluten peptides/proteins contained in urine samples.

Another object of the invention is constituted by the particular use of immunological methods based on monoclonal antibodies G12, A1 and R5 conjugated to an enzyme which allows quantitative assay using chromogenic, fluorogenic or luminescent substrates. The detection of the antigen-antibody bond could be carried out by means of substrates that give rise to soluble coloured products which can be measured in a spectrophotometer or that give rise to insoluble coloured products that form a precipitate at the antibody site, substrates that produce light that is detected, for example, using a luminometer or photographic film, biotin, subsequently detected by avidin/streptavidin (attached to a marker), fluorochromes, such as for example fluorescein and rhodamine, etc. This process could use a gliadin pattern, hydrolysed gliadin, the full 33-mer peptide (SEQ ID NO: 1) or part of its sequence of at least eight amino acids.

As will be understood by a person skilled in the art, the detection and/or quantification of the gluten ingested by a human being requires the definition of a calibration curve that will allow the analysis of the data obtained in the biological fluid in order to conclude whether or not an individual has ingested gluten and the amount of gluten ingested.

Therefore, in a particular embodiment, the detection and/or quantification of the gluten ingested by a human being comprises the definition of a calibration curve using peptides comprising the sequence SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or their deaminated derivatives.

In another particular embodiment, the detection and/or quantification of the gluten ingested by a human being comprises the definition of a calibration curve using pepsin-trypsinated gluten prolamines.

The invention proposes the detection of the gluten ingested by an individual by quantifying the immunogenic gluten peptides excreted in their urine and in other human body fluids such as saliva, sweat or serum.

The invention proposes an analytical control method for verifying GFD adherence and also for ruling out the uncontrolled ingestion of gluten by patients suspected of suffering so-called RCD.

Therefore, in a particular embodiment, the human being follows a GFD. Thus, based on this characteristic of human beings, the detection of gluten by means of the process of the invention is indicative that the human being is failing to follow the GFD.

Applying the method to verify the effectiveness of therapeutical strategies that reduce or eliminate gluten toxicity by means of enzymatic gluten detoxification (for example, Alvine Pharmaceuticals Ltd., USA; or DSM, The Netherlands), through the sequestration of the toxic prolamins by polymers to avoid their hydrolysis (for example, BiolineRX, Israel) or other alternative therapies forms part of the invention. The urine samples of coeliac patients subjected to clinical assays or to a therapeutic prescription in the future could also be controlled, as the effectiveness of the therapy could be determined by measuring the presence or absence of peptides in urine in the hours following the ingestion of a controlled amount of gluten, together with therapies that eliminate immunotoxic peptides, or after the ingestion of foods treated with a methodology that prevents the internalisation of immunotoxic peptides in the individual.

The current lack of consensus in clinical practice on how to control GFD and the determination of cases of involuntary exposure to gluten by contamination of the food items could be resolved by performing simple immunological assays of the urine samples. Healthcare professionals may consider these methods useful for monitoring coeliac patients and in the design of future clinical assays in order to draw conclusions consistent with the status of the patient's disease.

In another particular embodiment, the human being follows a therapy destined to avoid the ingestion of gluten and/or prevent the formation of immunogenic gluten peptides. In this context, the detection of gluten using the process of the invention is indicative that the therapy applied to said human being is not effective.

As a person skilled in the art understands, prior to bringing the biological fluid from the human being into contact with the antibody, the peptides found in the biological fluid can be concentrated.

In a particular embodiment of the process of the invention, a pre-concentration of the peptides is performed in the urine samples by means of a solid-phase extraction system (SPE), by the reversible physicochemical bonding of the peptides to the matrix, and subsequent unbonding thereof after elution with a solution of an adequate composition that will allow the detachment of the peptides from the matrix. It can also be concentrated by interaction with specific antibodies immobilised in chromatographic resins or in magnetic particles.

Subsequently, the concentrated polypeptides of gluten are eluted using a smaller volume of an adequate solution compatible with the subsequent quantification by means of immunological methods, for example immunochromatographic strips, that use at least one prolamin anti-peptide antibody, preferably those recognised by, but not limited to, the aforementioned A1, G12 and R5 antibodies. In order to quantify the amount of peptide, the process could be completed using reference patterns, or a standard pattern, with intact gluten proteins or, preferably, gluten polypeptides hydrolysed by pepsin and trypsin or synthesised. The signal intensity of known amounts of peptide standard would serve to extrapolate the amount of peptide in the sample, defining a calibration curve.

As is understood by the person skilled in the art, it is possible for the method of the invention to be performed in vivo in a human being, which is also envisaged in the present invention.

On the other hand, the present invention also envisages uses of kits for putting the process of the invention into practice. Thus, in another aspect, the present invention relates to a kit for detecting gluten in biological fluids, hereinafter, kit of the invention, comprising:
a) a standard pattern selected from
   - a peptide pattern comprise the sequence SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or their deaminated derivatives, and
   - a standard pattern of pepsin-trypsinated gluten prolamins, and
b) at least one antibody that specifically recognises gluten protein epitopes selected from among the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or their deaminated derivatives.

As indicated earlier, the process of the invention may comprise a stage of concentration of the peptides present in the biological fluid from the human being. To this end, a medium or matrix which allows the physicochemical bonding of the peptides found in the fluid and, subsequently, elution thereof using a buffered aqueous solution may be used. Alternatively, they may also be concentrated by interaction with specific antibodies immobilised in the chromatographic resins or in magnetic particles.

Therefore, in another particular embodiment, the kit comprises a solid peptide-bonding medium and/or an aqueous buffered reaction solution. These components allow the concentration of the peptides found in the biological fluid for the subsequent analysis thereof.

In another particular embodiment of the kit the antibody is selected from the group consisting of the monoclonal antibody G12, the monoclonal antibody A1 and the monoclonal antibody R5 which, in another particular embodiment, the antibody is comprised within an immunochromatographic strip which, in another particular embodiment, the antibody is bonded to an enzyme.

Throughout the description and claims, the word "comprises" and its variants do not aim to exclude other technical characteristics, additives, components or steps. For persons skilled in the art, other objects, advantages and characteristics of the invention shall be inferred partly from the description and partly from the practice of the invention. The following examples and figures are provided by way of illustration and do not aim to limit the present invention.

### BRIEF DESCRIPTION OF THE FIGURES

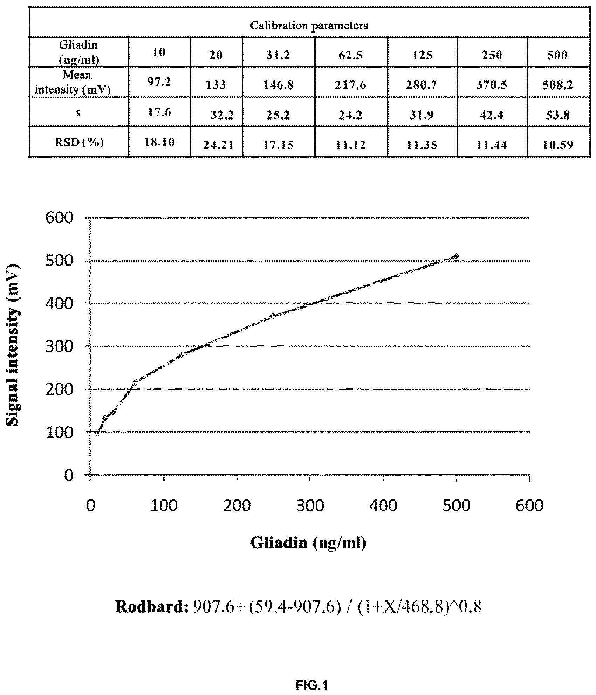

Figure 1. Relationship between the concentration of gliadin patterns and the intensity of the quantitative signal of the QI strip reader. The average value of the intensity, standard deviation(s) the relative standard deviation (RSD) was calculated for each standard pattern. The Rodbard function was calculated using the Reader software with the parameters included in the table.

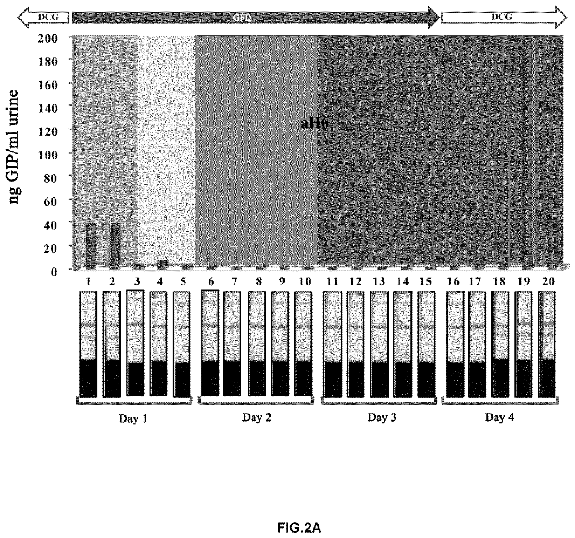

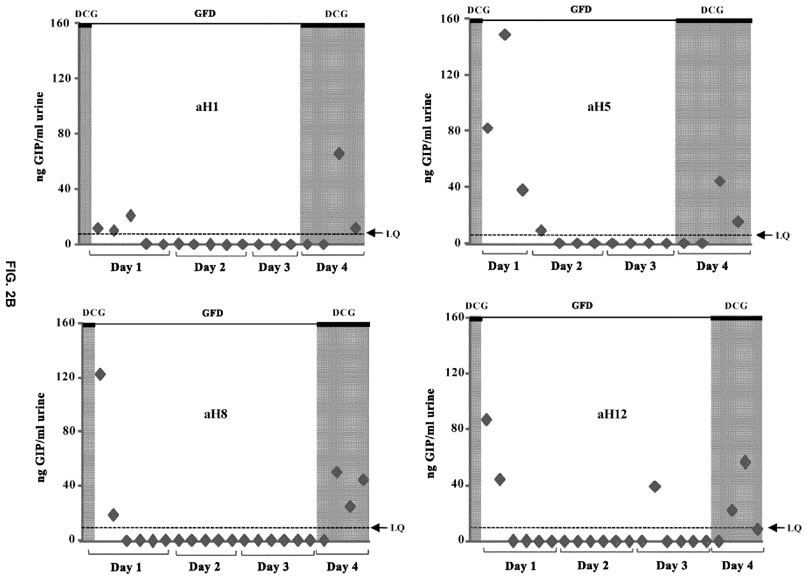

Figure 2A and 2B. Kinetics of elimination and appearance of GIP in urine samples of healthy individuals (n=13) subjected to a controlled gluten diet. Semi-quantification of gluten peptides/proteins by means of immunochromatographic strips with G12 antibody in the urine samples of healthy individuals after consuming a GCD, were subjected to a GFD until the reactive gluten peptides became undetectable. Afterwards, they were subjected to a GCD to verify the appearance of GIP in the urine samples again. Between three and six urine samples per day were collected from each individual for four days (Figure 2A) Example representative of the collection of IC-strips reacting with urine samples from an individual who participated in the study (aH6). The blue band is a positive internal control which indicates that the device has functioned correctly and the pink band indicates the presence of gluten peptides. Figure 2B) Kinetics of GIP excretion of four healthy individuals (aH1-aH2). GIP, gluten immunogenic peptides; GCD, gluten containing diet; GFD, gluten-free diet.

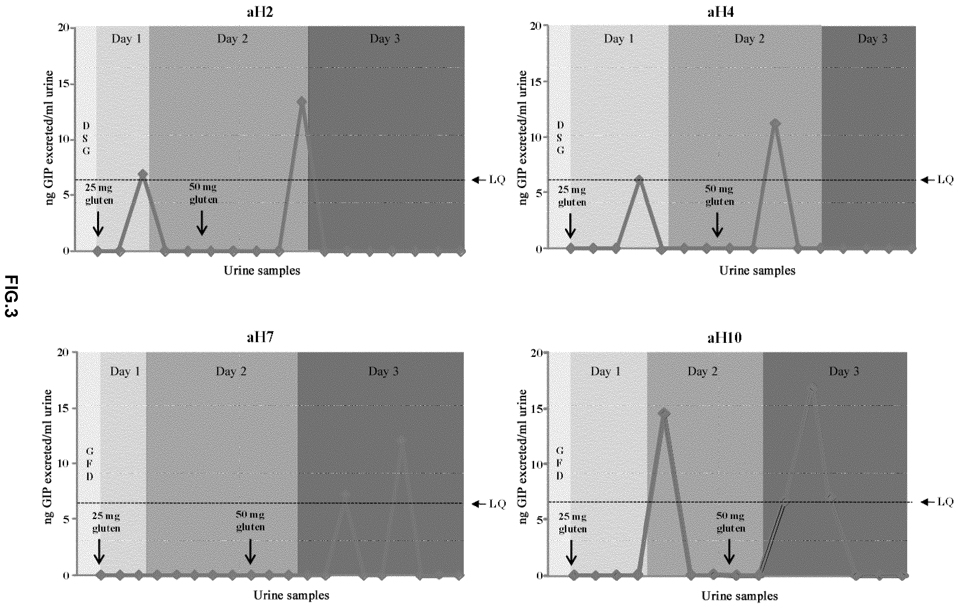

Figure 3. In vivo monitoring of the urinary excretion of the gluten-derived peptides of healthy individuals after controlled gluten ingestion. Three doses of gluten were administered (10, 25 and 50 mg) to healthy individuals who had consumed a GFD for one week. Four independent experiments corresponding to samples measured in triplicate for ingestions of 25 and 50 mg of gluten of four healthy individuals are shown. GIP, gluten immunogenic peptides; GFD, gluten-free diet.

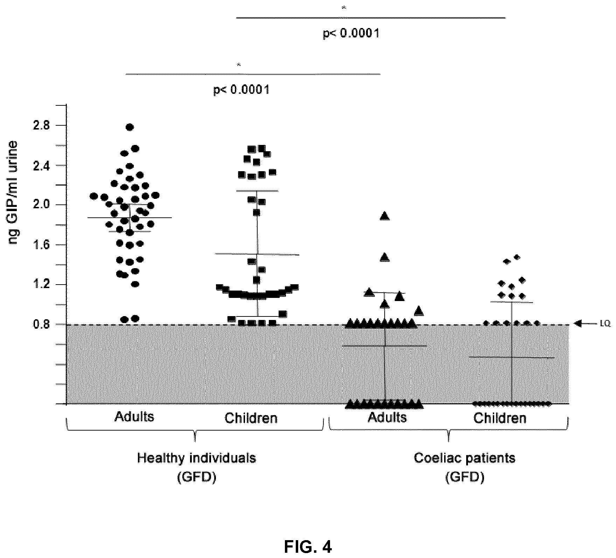

Figure 4. Monitoring of GFD adherence of coeliac patients through the detection and quantification of GIP in urine samples. The coeliac patients included in the study followed a GFD > 2 years and the healthy individuals who participated as positive controls consumed a GFD. The participants were divided into two groups according to age: adults (>16) and children (0-15). Each point represents the mean absorbance value (OD = 450 nm) of the analysed urine sample of each individual. In accordance with the quantification limit of the technique (QL = 0.8 ng/ml, indicated line), individuals with a GIP value greater than or equal to QL were considered positive for the presence of GIP, while those with GIP content lower than the QL were considered negative. *p <0.0001 (Student's t test). GIP, gluten immunogenic peptides; GFD, gluten-free diet; GCD, gluten containing diet; QL, quantification limit.

### EXAMPLES

### Example 1. Concentration, detection and semi-quantification of immunogenic gluten peptides in urine using immunochromatographic strips.

The present embodiment shows how immunogenic gluten peptides can be quantified in urine, despite the expected gastrointestinal digestion, after a pre-concentration process. Gluten is a complex mixture of proteins comprising gliadins and glutenins in wheat and equivalent proteins in barley, rye and some oat varieties. Gliadins and glutenins have a unique amino acid composition with high proline content (15%), hydrophobic amino acids (19%) and glutamine (35%); therefore, they form a resistant structure to complete digestion by pancreatic proteases. After the recent demonstration that alpha-gliadin 33-mer immunochromatographic strips, based on antibodies G12, A1 and R5, can detect hydrolyzed gluten peptides in faeces and beer samples with a high degree of sensitivity (Morón et al., 2008; Real et al., 2014; Osman et al., 2001), the question was raised as to whether or not gluten peptides were also excreted in urine.

Firstly, an assay was carried out in which urine samples of healthy individuals who followed a normal gluten-containing diet (GCD) (n=10) were collected. Given that gluten peptides are expected to be present in urine in very low concentrations, we are considering carrying out a pre-concentration stage that possibly also eliminates potential interference material in order to improve the possibilities of detecting GIP. The voluntary individuals were divided into two groups: one group (n=10) received a non-standardised diet which included the daily consumption of gluten and another group (n=10) was subjected to a GFD for one week. Written consent was obtained from the participating individuals. The following protocol was carried out for the purpose of the study:
- **Diet:** All the subjects who participated in the study were instructed to adhere to the established diet. Thus, those who consumed a GCD received instructions on the gluten-containing diet and those who consumed a GFD received instructions as to permitted foods. At the end of the study, adherence to the dietary conditions was determined through a structured interview.
- **Collection of urine samples:** Urine samples were collected from the subjects who participated in the study in sterile Falcon tubes and subsequently bottled and stored at -20°C until analysis thereof. All the samples were homogenised and aliquoted less than three hours after micturition.
- **Peptide concentration and cleaned-up of urine samples:** The SPE technique was used to concentrate the peptides and remove impurities from the urine samples collected. The octadecylsilane cartridges used were previously conditioned with 1 ml of formic acid at 0.1% in 80% methanol through the application of a vacuum and the eluent was discarded. Next, 7.5 ml of trifluoroacetic acid (TFA) were added and, after the vacuum, the eluent was discarded. Separately, a mixture of 5 ml of 50% of urine in TFA was centrifuged for 10 min at 2,500 g and the concentrated peptides of interest were eluted with 1 ml of PBS buffer solution compatible with Glutentox-strips.

After concentrating the urine samples of all the healthy individuals (n=20) subsequently using SPE cartridges, GIP detection was carried out in the urine samples of the individuals included in the two different diet groups using immunochromatographic strips Glutentox Sticks (Biomedal, S.L., Seville, Spain). The gluten peptides were detected in all the concentrated urines of GCD subjects. However, toxic gluten peptides were not detected in any concentrated urine of GFD subjects. These results indicate that the signal was dependent on gluten ingestion.

In order to correlate GIP concentration and the output signal of the immunochromatigraphic strips analysed, the strip reader was calibrated using an alpha-gliadin 33-mer synthetic peptide standard and the urine of a patient who had been following a GFD for more than two years and whose serum markers were analysed to confirm the patient's strict adherence to the diet. In order to verify the negative ingestion of gluten by the patient, a qualitative analysis was performed using anti-GIP Glutentox-strips containing G12 antibodies, in urine and faeces concentrates in accordance with the protocol established by Comino et al. (2012). A series of five 33-mer peptide standards at 3.12, 6.25, 12.5, 25, 50, 75, 100 and 200 ng/ml was prepared and added to the control urine, collecting the value of the peak height of the line of the IC-strips which was evaluated using Glutentox Reader® (Biomedal S.L., Seville, Spain). The mean value was calculated in each standard, as well as its standard deviation (SD) and relative standard deviation (RSD). The mean values were used to calculate the calibration function, which is adjusted to a Rodbard function (Figure 1). Next, the calibration function was entered in the anti-GIP IC-strip reader software to quantify the GIP in the urine samples. The quantification limit (QL) of the technique was established at 6.48 ng GIP/ml urine.

Once the viability of the method for detecting gluten in urine was confirmed, an assay was conducted for the purpose of semi-quantifying the level of GIP in the urine samples. To this end, an assay was conducted in which it was verified by dietary interview that gluten had been consumed by the healthy individuals participating in the study (n=10, 7 women and 3 men with an average age of 23-39). The inclusion criteria comprised the absence of diseases, digestive symptoms, drugs, antibiotics in the last two months and absence of family history of CD. All the participants were tested for CD, obtaining normal serum tTG levels and their HLA-DQ phenotype was not DQ-2/- 8. The haemoglobin levels and biochemical blood analysis, including kidney and liver tests, were found to be included in the range of normal values. This study was approved by the local Ethics Committee of the "Hospital Virgen de Valme" (Seville, Spain). The written consent of the participating individuals was obtained. Urine samples were collected from individuals following a normal gluten-containing diet (bread, pasta, biscuits, etc.). The presence of gluten-derived peptides in the concentrated urine extracts was determined using G12 antibody-based immunochromatographic strips (IC, Glutentox Sticks) by reading signal intensity using Glutentox Reader®. The samples were immersed in the dilution solution proposed by the manufacturer. The immunochromatographic strips were immersed in the different samples (300 µl) for 10 minutes and left to air dry. In this case, the excretion of gluten peptides in the urine of all the individuals recorded values greater than 20 ng/ml.

### Example 2. Monitoring of immunogenic gluten-derived peptides in urine samples of individuals following a controlled gluten-containing diet.

This example shows how the elimination and appearance time of ingested gluten in urine samples can be determined using G12 antibody-based IC-strips. To this end, a total of 13 healthy volunteers (9 women and 4 men, with an average age of 23-65) were subjected to different dietary conditions. Urine samples were collected from those individuals who consumed a GCD and were subjected to a gluten-free diet (GFD) until the GIP level became undetectable in the urine samples. Afterwards, a GCD was re-introduced and new urine samples were collected. A total of 3-6 different urine samples of each individual were collected each day. Figure 2A shows a representative example of the collection of IC-strips reacting with urine samples collected during the study period of a healthy subject (AH6). The kinetics of GIP excretion of 4 of the 13 healthy volunteers (AH1, AH5, AH8 and AH12) analysed reveal that the gluten consumed was completely eliminated between 16 and 34 hours after commencing the GFD in all the assayed individuals (Figure 2B).

After re-introducing gluten in the diet, GIP were detected in urine samples after 3 to 9 hours. The differences in excretion observed between individuals could be related to diet, type of gluten-containing foods, and variability of the hydrolitic capacity of the digestive system (mainly enzymes) and microbial activities. In 12 of the 13 healthy individuals in GFD, the amounts of GIP in urine were lower than the QL of the method. The other patients initially had good adherence to the GFD; however, a signal of 40 ng GIP/ml urine was detected on the third day of the assay, as shown in the graph of individual AH12. On being interviewed at the end of the study, this individual confirmed the involuntary consumption of yoghurt with cereals (wheat, among others) a few hours before one of the urine sample collections.

After consuming gluten, a high degree of variability in the concentration and time of excretion of reactive gluten peptides was observed between individuals. Upon confirming the viability of the method for detecting gluten in urine, we studied whether or not there was correlation between the amount of gluten ingested and the amount of GIP measured in the urine using this methodology. In accordance with recent systematic reviews, a total daily consumption of gluten of less than 10-50 mg can cause histological abnormalities (Hischenhuber et al, 2006; Catassi et al, 2007; Akobeng y Thomas, 2008; Gibert et al., 2013). In order to verify whether or not the anti-GIP LFT methodology was capable of detecting a low consumption of gluten in urine samples, certain amounts of ingested gluten were established (10-50 mg), which were administered to four healthy individuals (Figure 3). A single type of gluten was used in all cases. The individuals were given a standard piece to exclude variability due to the administration of gluten from different sources. An initial microdosis of 10 mg of gluten was administered and the gluten content in the urine samples collected was measured using anti-GIP LFT. Next, doses of 25 and 50 mg were administered and GIP amounts in urine were also measured until the reactive gluten peptides became undetectable. After consuming an amount of food equivalent to 50 mg of gluten, GIP were detected in the urine of all the individuals analysed and, therefore, the detection limit (DL) of this method is 50 mg of gluten. However, the administration of 25 mg of gluten produced measurable signals in the IC-strips after analysing the urine of three of the four persons analysed (AH2, AH4 and AH10), although the signal was only clearly higher than the QL in AH10. There were no detectable signals after ingestion of 10 mg in any of the individuals. The individual variations observed were probably due to the individual differences in metabolism and diets, the intestinal microbiota and variations in the daily consumption of foods.

### Example 3. Monitoring of adherence to the gluten-free diet in urine samples of coeliac patients.

This example shows how GFD adherence of coeliac patients can be monitored through the determination of immunogenic gluten peptides in urine using a fast detection system based on IC-strips with the G12 antibody (Glutentox Sticks, Biomedal, S.L.). The high percentage of coeliac patients with partial recovery of the intestinal mucosa, probably due to involuntary ingestions, has given rise to the need for a non-invasive marker which allows short-term monitoring of GFD adherence. The passage of gluten through the gastrointestinal tract gives rise to the hydrolysis of the greater part thereof. In order to assess the adequacy of the method for monitoring gluten ingestion in coeliac patients, the urine samples of 76 healthy individuals were studied (42 adults and 34 children) who followed a GCD and 58 coeliac patients in remission (27 adults and 31 children) who had been subjected to GFD for > 2 years.

The urine samples of healthy individuals after consuming a gluten-containing diet had, in all cases, GIP levels higher than the QL of the method (76 out of 76, 100% positive in GIP in urine). The range of urine GIP values in healthy individuals varied, being 6.54 to 604 ng/ml urine in adults and 6.48 to 369 ng/ml urine in children, i.e. between 57 and 93 times higher than the QL of the method, which suggests that the excretion of gluten peptides is strongly affected by diet, types of gluten-containing foods and/or individual characteristics such as intestinal microflora. When the assay was conducted in the urine of coeliac patients, the urine GIP level was less than the QL of the method only in 41% of adults and in 55% of children with CD in clinical remission. Clear GFD infringement was observed in the other coeliac individuals, in whom GIP content ranged between 4.5 and 12 times higher than the QL (6.48-78.12 and 6.48-29.69 ng GIP/ml urine, adults and children, respectively).

### Example 4. Monitoring of gluten-free diet in urine samples collected over a 24-hour period in individuals subjected to gluten-containing or gluten-free diets.

This example shows how GFD can be monitored in all the urine samples collected over a 24-hour period using an immunochromatographic reading system with anti-GIP antibodies. The substances eliminated by the kidneys are not excreted at the same speed or in the same amounts at different time intervals during the day or during the night; under normal excretion conditions, less urine is eliminated during the night than during the day. Therefore, a random urine sample may not generate an image integrated by possible specific gluten ingestions over a 24-hour period. The analysis of 24-hour urine samples, in addition to the serum studies, was used in general to obtain relevant data in many disorders.

Therefore, the objective of this study was to determine the capacity of this technique to measure the gluten-derived peptides in 24-hour urine as an indicator of GFD adherence in coeliac patients. To this end, three healthy individuals who followed a GCD and one coeliac patient in remission with GFD > 2 years participated in the study. All the urine samples of four adult participants excreted over a 24-hour period were collected separately. Each urine sample was analysed separately to determine its gluten content and, afterwards, all the urine samples were confined in a single receptacle. The detection and quantification of GIP by IC-strips was performed in the four 24-hour samples. The three healthy individuals showed GIP levels in both the individual urine samples and the 24-hour sample, wherein the level of gluten peptides ranged from 12.4 to 87.6 ng/ml. However, the GIP levels in the urine samples of the coeliac patient were lower than the QL of the method and in the 24-hour sample. The GIP measurements obtained in discontinuous urine samples were very similar to those obtained in complete 24-hour samples. Therefore, the results indicated that the amount of gluten in 24-hour urine provides valuable information on the mean value of the metabolism and excretion of gluten consumed over a complete 24-hour period in an individual.

### Example 5. Monitoring of gluten-free diet in urine samples using a biosensor.

This example shows how it is possible to determine the amount of GIP in urine using a biosensor. It is a selective and quantitative technique for directly detecting gluten-derived peptides without need for markers. To this end, an anti-GIP antibody is immobilised on a 100 nm-sized hydrophobic matrix with 2% dextran. The buffer in which the antibody is diluted must favour the electrostatic interaction between the anti-GIP antibody and the dextran matrix in order to facilitate the formation of links. In an aqueous medium, the pKa of the matrix groups is approximately 4. Antibody immobilisation concentrations range between 30 and 130 µM, although they depend on the anti-GIP used. This immobilisation generates a resonance signal that is collected by the detector and is called basal signal. Next, the urine is conducted over the sensor chip under study. If it contains GIP, a change in signal is generated in the detector, which is a response directly proportional to the interaction between the GIPs and the antibody. Lastly, the surface is washed to remove residual urine, leaving the anti-GIP attached. In this manner, the surface is ready for a new assay.

### Example 6. Monitoring of the gluten-free diet in urine samples by means of a system of paramagnetic and chemiluminescent particles.

This example shows how the presence of gluten peptides in urine can be determined using an anti-GIP antibody-coated particle. An antibody is immobilised against an immunogenic gluten peptide, G12, in 0.2 micron-sized magnetic particles. Approximately 0.1 ml of magnetic particles with a minimum concentration of 107 particles/ml is brought into contact with 2 ml of urine to capture the peptides contained therein in an Eppendorf tube. The process of washing and binding the particles to the walls of the tube is performed by means of the usual processes used by technical experts in the field using a magnet adapted to the tube (Life Technologies, Miltenji, Sepmag). An anti-GIP antibody conjugated to peroxidase that must only be bonded to those particles which have detected a GIP with two epitopes. After washing using binding/unbinding from the walls of the magnet-dependent tube, it is revealed by the application of luminal and a luminometer is used to quantify the signal. A signal higher than the negative control of the sample without GIP would indicate the presence of gluten peptides.

## Claims

1. Use of an immunological method for detecting and/or quantifying in a urine sample the gluten ingested with the food diet by a human being, wherein the immunological method comprises at least one antibody that specifically recognises gluten protein epitopes selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or their deaminated derivatives, wherein the recognition of the gluten protein epitopes by the antibodies is indicative that the human being has ingested gluten.

2. An *in vitro* process for detecting and/or quantifying the gluten ingested with the food diet by a human being, which comprises bringing an urine sample from said human being into contact with at least one antibody that specifically recognises gluten protein epitopes selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or their deaminated derivatives, wherein the recognition of the epitopes by the antibodies is indicative that the human being has ingested gluten.

3. Process according to claim 1, wherein the antibody is selected from the group consisting of the monoclonal antibody G12, the monoclonal antibody A1 and the monoclonal antibody R5.

4. Process according to claim 2 or 3, wherein the biological fluid is brought into contact with the antibodies by means of an immunological method selected from the group consisting of immunochromatographic strips, fluorescent immunoparticles, magnetic immunoparticles, indirect ELISA, competitive ELISA, sandwich ELISA, Western blot, electrochemical biosensors, plasmon resonance biosensors, thermoelectric biosensors and nanomechanical biosensors.

5. Process according to claim 3 or 4, wherein the monoclonal antibody G12, monoclonal antibody A1 or monoclonal antibody R5 is conjugated to molecules which allow quantitative assay by means of densitometry, fluorimetry, luminescence or electrochemical reaction.

6. Process according to claim 5, wherein the molecules which allow a quantitative assay are selected from the group consisting of a chromogenic substrate, a fluorogenic substrate and a luminescent substrate.

7. Process according to any one of claims 2 to 6, wherein the peptides are concentrated by means of solid-phase extraction prior to bringing the biological fluid into contact with the antibodies.

8. Process according to any one of claims 2 to 7, wherein the human being follows a gluten-free diet.

9. Process according to claim 8, wherein the detection of gluten is indicative that the human being is failing to follow the gluten-free diet.

10. Process according to any of claims 2 to 9, wherein the human being follows a therapy aimed at avoiding gluten ingestion and/or avoiding the formation of immunogenic gluten peptides.

11. A process, according to claim 10, wherein the detection of gluten is indicative that the therapy applied to said human being is not effective.

12. Use of a kit comprising
a) a standard pattern selected from
- a peptide pattern comprising the sequence SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or their deaminated derivatives, and
- a standard pepsin-trypsinated gluten prolamin pattern, and
b) at least one antibody that specifically recognises gluten protein epitopes selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 and/or their deaminated derivatives, for putting into practice a process according to nay of claims 2 to 11.

13. Use of a kit according to claim 12, wherein the antibody is selected from the group consisting of the monoclonal antibody G12, the monoclonal antibody A1 and the monocolonal antibody R5.

## Patentansprüche

1. Verwendung eines immunologischen Verfahrens zum Nachweis und/oder zur Quantifizierung des von einem Menschen mit der Nahrungsaufnahme aufgenommenen Glutens in einer Urinprobe, wobei das immunologische Verfahren mindestens einen Antikörper umfasst, der spezifisch Glutenproteinepitope erkennt, die ausgewählt sind aus der Gruppe, bestehend aus SEQ ID NO:2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 und/oder deren deaminierte Derivate, wobei die Erkennung der Glutenproteinepitope durch die Antikörper anzeigt, dass der Mensch Gluten aufgenommen hat.

2. *In vitro*-Prozess zum Nachweis und/oder zur Quantifizierung des von einem Menschen mit der Nahrungsaufnahme aufgenommenen Glutens, der das Inkontaktbringen einer Urinprobe des Menschen mit mindestens einem Antikörper umfasst, der spezifisch Glutenproteinepitope erkennt, die ausgewählt sind aus der Gruppe, bestehend aus SEQ ID NO:2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 und/oder deren deaminierte Derivate, wobei die Erkennung der Epitope durch die Antikörper anzeigt, dass der Mensch Gluten aufgenommen hat.

3. Prozess nach Anspruch 1, wobei der Antikörper ausgewählt ist aus der Gruppe, bestehend aus dem monoklonalen Antikörper G12, dem monoklonalen Antikörper A1 und dem monoklonalen Antikörper R5.

4. Prozess nach Anspruch 2 oder 3, wobei das biologische Fluid mittels eines immunologischen Verfahrens in Kontakt mit den Antikörpern gebracht wird, das ausgewählt ist aus der Gruppe, bestehend aus immunochromatographischen Streifen, fluoreszierenden Immunopartikeln, magnetischen Immunopartikeln, indirektem ELISA, kompetitivem ELISA, Sandwich-ELISA, Western Blot, elektrochemischen Biosensoren, Plasmonresonanz-Biosensoren, thermoelektrischen Biosensoren und nanomechanischen Biosensoren.

5. Prozess nach Anspruch 3 oder 4, wobei der monoklonale Antikörper G12, der monoklonale Antikörper A1 oder der monoklonale Antikörper R5 an Moleküle konjugiert sind, die eine quantitative Bestimmung mittels Densitometrie, Fluorimetrie, Lumineszenz oder elektrochemischer Reaktion ermöglichen.

6. Prozess nach Anspruch 5, wobei die Moleküle, die eine quantitative Bestimmung ermöglichen, ausgewählt sind aus der Gruppe, bestehend aus einem chromogenen Substrat, einem fluorogenen Substrat und einem Lumineszenzsubstrat.

7. Prozess nach einem der Ansprüche 2 bis 6, wobei die Peptide mittels Festphasenextraktion konzentriert werden, bevor das biologische Fluid mit den Antikörpern in Kontakt gebracht wird.

8. Prozess nach einem der Ansprüche 2 bis 7, wobei der Mensch eine glutenfreie Ernährung befolgt.

9. Prozess nach Anspruch 8, wobei der Nachweis von Gluten anzeigt, dass der Mensch die glutenfreie Ernährung nicht befolgt.

10. Prozess nach einem der Ansprüche 2 bis 9, wobei der Mensch einer Therapie folgt, die darauf abzielt, die Aufnahme von Gluten und/oder die Bildung von immunogenen Glutenpeptiden zu vermeiden.

11. Prozess nach Anspruch 10, wobei der Nachweis von Gluten anzeigt, dass die an den Menschen angewendete Therapie nicht wirksam ist.

12. Verwendung eines Kits, umfassend
a) ein Standardmuster, ausgewählt aus
- einem Peptidmuster, umfassend die Sequenz SEQ ID NO:2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 oder deren deaminierte Derivate, und
- ein Standard-Pepsin-Trypsin-Gluten-Prolamin-Muster und
b) mindestens einen Antikörper umfasst, der spezifisch Glutenproteinepitope erkennt, die ausgewählt sind aus der Gruppe, bestehend aus SEQ ID NO:2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 und/oder deren deaminierte Derivate zum Umsetzen eines Prozesses nach einem der Ansprüche 2 bis 11 in der Praxis.

13. Verwendung eines Kits nach Anspruch 12, wobei der Antikörper ausgewählt ist aus der Gruppe, bestehend aus dem monoklonalen Antikörper G12, dem monoklonalen Antikörper A1 und dem monoklonalen Antikörper R5.

## Revendications

1. Utilisation d'une méthode immunologique pour la détection et/ou la quantification dans un échantillon d'urine du gluten ingéré par l'homme dans le régime alimentaire, dans laquelle la méthode immunologique comprend au moins un anticorps qui reconnaît spécifiquement les épitopes des protéines du gluten choisis dans le groupe consistant en SEQ ID N ° 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 et/ou leurs dérivés désaminés, dans laquelle la reconnaissance des épitopes des protéines de gluten par les anticorps indique que l'homme a ingéré du gluten.

2. Procédé *in vitro* de détection et/ou de quantification du gluten ingéré par l'homme dans le régime alimentaire, qui comprend la mise en contact d'un échantillon d'urine dudit homme avec au moins un anticorps qui reconnaît spécifiquement les épitopes des protéines de gluten choisis dans le groupe consistant en SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 et/ou leurs dérivés désaminés, dans lequel la reconnaissance des épitopes par les anticorps indique que l'homme a ingéré du gluten.

3. Procédé selon la revendication 1, dans lequel l'anticorps est choisi dans le groupe consistant en l'anticorps monoclonal G12, l'anticorps monoclonal A1 et l'anticorps monoclonal R5.

4. Procédé selon les revendications 2 ou 3, dans lequel le fluide biologique est mis en contact avec les anticorps au moyen d'une méthode immunologique choisie dans le groupe consistant en bandelettes immunochromatographiques, immunoparticules fluorescentes, immunoparticules magnétiques, test ELISA indirect, test ELISA par compétition, test ELISA en sandwich, buvardage de western, biocapteurs électrochimiques, biocapteurs à résonance plasmonique, biocapteurs thermoélectriques et biocapteurs nanomécaniques.

5. Procédé selon les revendications 3 ou 4, dans lequel l'anticorps monoclonal G12, l'anticorps monoclonal A1 ou l'anticorps monoclonal R5 est conjugué à des molécules permettant un dosage quantitatif par densitométrie, fluorimétrie, réaction de luminescence ou électrochimique.

6. Procédé selon la revendication 5, dans lequel les molécules permettant un dosage quantitatif sont choisies dans le groupe consistant en un substrat chromogénique, un substrat fluorogène et un substrat luminescent.

7. Procédé selon l'une quelconque des revendications 2 à 6, dans lequel les peptides sont concentrés au moyen d'une extraction en phase solide avant la mise en contact du fluide biologique avec les anticorps.

8. Procédé selon l'une quelconque des revendications 2 à 7, dans lequel l'homme suit un régime sans gluten.

9. Procédé selon la revendication 8, dans lequel la détection de gluten indique que l'homme ne suit pas le régime sans gluten.

10. Procédé selon l'une quelconque des revendications 2 à 9, dans lequel l'homme suit une thérapie visant à éviter l'ingestion de gluten et/ou à éviter la formation de peptides de gluten immunogènes.

11. Procédé, selon la revendication 10, dans lequel la détection de gluten indique que la thérapie appliquée audit homme n'est pas efficace.

12. Utilisation d'un kit comprenant
a) un motif standard choisi parmi
- un motif peptidique comprenant la séquence SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 ou leurs dérivés désaminés, et
- un motif standard de prolamine de gluten trypsinisé avec de la pepsine et
b) au moins un anticorps qui reconnaît spécifiquement les épitopes des protéines de gluten choisis dans le groupe consistant en SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 et/ou leurs dérivés désaminés, pour la mise en œuvre d'un procédé selon l'une quelconque des revendications 2 à 11.

13. Utilisation d'un kit selon la revendication 12, dans laquelle l'anticorps est choisi dans le groupe consistant en l'anticorps monoclonal G12, l'anticorps monoclonal A1 et l'anticorps monoclonal R5.


### SEQUENCE LISTING

<110> BIOMEDAL S.L.
   UNIVERSIDAD DE SEVILLA
<120> DETECTING GLUTEN PEPTIDES IN HUMAN FLUIDS
<130> PCT3007.5
<150> ES P201400569
   <151> 2014-07-09
<160> 11
<170> PatentIn version 3.5
<210> 1
   <211> 33
   <212> PRT
   <213> Triticum aestivum
<400> 1
   Leu Gln Leu Gln Pro Phe Pro Gln Pro Gln Leu Pro Tyr Pro Gln Pro
   1               5                   10                  15
   Gln Leu Pro Tyr Pro Gln Pro Gln Leu Pro Tyr Pro Gln Pro Gln Pro
               20                  25                  30
   Phe
<210> 2
   <211> 6
   <212> PRT
   <213> Triticum aestivum
<400> 2
   Gln Pro Gln Leu Pro Tyr
   1               5
<210> 3
   <211> 6
   <212> PRT
   <213> Triticum aestivum
<400> 3
   Gln Pro Gln Leu Pro Phe
   1 5
<210> 4
   <211> 6
   <212> PRT
   <213> Triticum aestivum
<400> 4
   Gln Pro Gln Leu Pro Leu
   1 5
<210> 5
   <211> 7
   <212> PRT
   <213> Triticum aestivum
<400> 5
   Gln Gln Pro Phe Pro Gln Pro
   1 5
<210> 6
   <211> 7
   <212> PRT
   <213> Triticum aestivum
<400> 6
   Gln Leu Pro Tyr Pro Gln Pro
   1 5
<210> 7
   <211> 7
   <212> PRT
   <213> Triticum aestivum
<400> 7
   Gln Leu Pro Phe Pro Gln Pro
   1 5
<210> 8
   <211> 7
   <212> PRT
   <213> Triticum aestivum
<400> 8
   Gln Gln Pro Tyr Pro Gln Pro
   1 5
<210> 9
   <211> 6
   <212> PRT
   <213> Triticum aestivum
<400> 9
   Gln Pro Gln Pro Leu Tyr
   1               5
<210> 10
   <211> 8
   <212> PRT
   <213> Triticum aestivum
<400> 10
   Phe Pro Leu Gln Pro Gln Gln Pro
   1               5
<210> 11
   <211> 8
   <212> PRT
   <213> Triticum aestivum
<400> 11
   Phe Pro Leu Glu Pro Glu Glu Pro
   1               5